# EUROPEAN PATENT SPECIFICATION

(11) **EP 2 794 902 B1**
(45) Date of publication and mention of the grant of the patent: **22.08.2018**
(21) Application number: 12858855.5
(22) Date of filing: 19.12.2012
(51) Int. Cl.: H01M 8/1009, H01M 8/06, H01M 8/16

(54) **PROCESSING BIOMASS FOR USE IN FUEL CELLS**
VERARBEITUNG VON BIOMASSE ZUR VERWENDUNG IN BRENNSTOFFZELLEN
TRAITEMENT DE BIOMASSE POUR UNE UTILISATION DANS DES PILES À COMBUSTIBLE

(30) Priority: 22.12.2011 US 201161579568 P
(43) Date of publication of application: 29.10.2014
(60) Divisional application: 18179356.3
(73) Proprietor: Xyleco, Inc., Wakefield, MA 01880 (US)
(72) Inventor: MEDOFF, Marshall, Woburn, Massachusetts 01801 (US); MASTERMAN, Thomas Craig, Woburn, Massachusetts 01801 (US)
(74) Representative: FRKelly
(86) International application number: PCT/US2012/070624
(87) International publication number: WO 2013/096452

(56) References cited:
- WO-A1-2011/090544
- JP-A- 2006 314 982
- US-A1- 2010 200 806
- US-A1- 2010 200 806
- US-A1- 2011 177 558
- THYGESEN A ET AL: "The effect of different substrates and humic acid on power generation in microbial fuel cell operation", BIORESOURCE TECHNOLOGY, ELSEVIER BV, GB, vol. 100, no. 3, 1 February 2009 (2009-02-01), pages 1186-1191, XP025645348, ISSN: 0960-8524, DOI: 10.1016/J.BIORTECH.2008.07.067 [retrieved on 2008-09-23]

## Description

### RELATED APPLICATIONS

This application claims priority to U.S. Provisional Application Serial No. 61/579,568, filed December 22, 2011.

### BACKGROUND

Cellulosic and lignocellulosic materials (e.g., biomass materials) are produced, processed, and used in large quantities in a number of applications. Often such materials are used once, and then discarded as waste, or are simply considered to be waste materials, e.g., sewage, bagasse, sawdust, and stover.

A typical biomass material contains cellulose, hemicellulose, and lignin plus lesser amounts of proteins, extractables and minerals. The complex carbohydrates contained in the cellulose and hemicellulose fractions can be processed into fermentable sugars by saccharification, using a cellulolytic enzyme, and the sugars can then be used as an end product or intermediate, or converted by further bioprocessing, e.g., fermentation, into a variety of products, such as alcohols or organic acids. The product obtained depends upon the microorganism utilized and the conditions under which the bioprocessing occurs.

JP-A 2006-314982 discloses a method of utilizing residues produced during a process of mass sulfuric acid saccharification of a woody/herbaceous biomass such as rice straw. The method comprises (1) using a saccharification liquor as-is for power generation using a glucose fuel cell, or to produce a useful material via inoculating and culturing the saccharification liquor, while simultaneously making use of a gypsum-containing biomass residue produced by solid-liquid separation following neutralization as a soil improver, turbidity suppressing agent, fine soil erosion suppressing agent, composting material, gypsum-containing construction material, or the like; (2), as in (1), wherein a fuel cell comprising xylose dehydrogenase immobilized on an electrode is used; (3), as in (1) or (2), wherein a strongly acidic biomass residue produced after sulfuric acid saccharification of the woody/herbaceous biomass is used as-is as an alkaline soil improver or alkaline waste material neutralizing agent; and (4), as in any of (1)-(3), wherein a residue or fermentation liquor produced by fermenting a liquid fraction obtained via solid-liquid separation into a valuable material, then separating/extracting the material, is used to produce a fertilizer, soil improver, probiotic, single-cell protein (SCP), or the like.

US 2010/0200806 A1 discloses the processing of biomass feedstocks (e.g., plant biomass, animal biomass, and municipal waste biomass) to produce useful products, such as fuels. For example, systems are described that can convert feedstock materials to a sugar solution, which can then be fermented to produce ethanol.

### SUMMARY

This invention relates to methods of processing (e.g., saccharifying) carbohydrate-containing materials (e.g., biomass materials or biomass-derived materials) to produce sugar (e.g., glucose) solutions that can be used in fuel cells such as direct sugar fuel cells, indirect sugar fuel cells and biological fuel cells. The invention also relates to utilizing the carbohydrate-containing material derived sugars solutions in fuel cells.

More specifically the invention relates to a method according to claim 1 wherein the fuel cell especially contains an anti-scale or an anti-fouling agent.

In some implementations, the sugar solution is produced by the saccharification of a lignocellulosic or cellulosic material, for example by contacting the material with an enzyme (e.g., a cellulase). In other implementations the recalcitrance of the lignocellulosic or cellulosic material has been reduced relative to that of the material in its native state prior to saccharification. In some cases, reducing the recalcitrance of the feedstock includes treating the feedstock with a treatment. The treatment can be, for example, ionization radiation (e.g., electron beam radiation), sonication, pyrolysis, oxidation, steam explosion, chemical treatment, various mechanical treatments and combinations of any of these treatments. The physical treatment can comprise any one or more of the treatments disclosed herein, applied alone or in any desired combination, and applied once or multiple times. When treating with ionizing radiation, e.g., electron beam radiation, the dosage can be at least 10 Mrad and less than about 200 Mrad (eg., 50 Mrad to 150Mrad).

In some implementations, the method can include using an additive in the fuel cell. For example additives can be acids, bases, buffers (e.g., phosphate buffers), minerals, colloids, emulsions, emulsifiers, particulates, nano-particles, cations, anions, metal ions (e.g., Fe²⁺, Fe³⁺, Mn2⁺, Cu2⁺, K⁺, Na⁺), vitamins, enzymes, peptones, extracts, surfactants, nutrients, gases (e.g., hydrogen, nitrogen, helium, argon, carbon monoxide, carbon dioxide), chemicals, nitrogen sources (e.g., ammonia, urea), pigments, fragrances, anionic polymers, cationic polymers, non-ionic polymers, oligomers, lipids, fats, surfactants, dispersants, anti-foam agents, bacteriostatic agents, antimicrobial agents, microorganisms, viscosity modifiers, oxidizing agents (e.g., peroxides, chlorates), reducing agents, corrosion inhibitors, precipitating agents, coagulants added in any combination and sequence.

A typical biomass resource contains cellulose, hemicellulose, and lignin plus lesser amounts of proteins, extractables and minerals. In some implementations, cellulosic or lignocellulosic material includes paper, paper products, paper waste, paper pulp, pigmented papers, loaded papers, coated papers, filled papers, magazines, printed matter, printer paper, polycoated paper, card stock, cardboard, paperboard, offal, cotton, wood, particle board, forestry wastes, sawdust, aspen wood, wood chips, grasses, switchgrass, miscanthus, cord grass, reed canary grass, grain residues, rice hulls, oat hulls, wheat chaff, barley hulls, agricultural waste, silage, canola straw, wheat straw, barley straw, oat straw, rice straw, jute, hemp, flax, bamboo, sisal, abaca, corn cobs, corn stover, soybean stover, corn fiber, alfalfa, hay, coconut hair, sugar processing residues, bagasse, beet pulp, agave bagasse, algae, seaweed, manure, sewage, arracacha, buckwheat, banana, barley, cassava, kudzu, oca, sago, sorghum, potato, sweet potato, taro, yams, beans, favas, lentils, peas, and mixtures of any of these.

The cellulosic or lignocellulosic material can be mechanically treated to reduce the bulk density of the cellulosic or lignocellulosic material and/or increase its surface area. In some implementations, the method includes mechanically treating the feedstock before and/or after reducing its recalcitrance. Mechanical treatments include, for example, cutting, milling, e.g., hammermilling, pressing, grinding, shearing and chopping. For example, comminuting the biomass material can be effective treatment applied to the biomass material. Mechanical treatment can reduce the bulk density of the feedstock and/or increase the surface area of the feedstock. In some embodiments, after mechanical treatment the material has a bulk density of less than 0.75 g/cm³, e.g., less than about 0.7, 0.65, 0.60, 0.50, 0.35, 0.25, 0.20, 0.15, 0.10, 0.05, or less, e.g., less than 0.025 g/cm³. Bulk density is determined using ASTM D1895B.

In one aspect, the invention features utilizing a sugar solution derived from a cellulosic or lignocellulosic material as described above in a fuel cell, e.g., a direct sugar fuel cell, an indirect sugar fuel cell and/or a biological fuel cell. Optionally the fuel can be a sugar or an alcohol derived from the saccharification of the cellulosic or lignocellulosic material.

Other features and advantages of the invention will be apparent from the following detailed description, and from the claims.

### DESCRIPTION OF DRAWINGS

FIG. 1 is a diagram illustrating the enzymatic hydrolysis of cellulose to glucose.
FIG. 2 is a flow diagram illustrating conversion of a biomass feedstock for use in a fuel cell.
FIG. 3 is a diagram showing various processes using saccharified feedstock in fuel cells.
FIG. 4 is a diagrammatic view of an example of a direct glucose fuel cell.
FIG. 5 is a diagrammatic view of an example of an indirect sugar fuel cell.
FIG. 6 is a diagrammatic view of a half-cell of a biological fuel cell.

### DETAILED DESCRIPTION

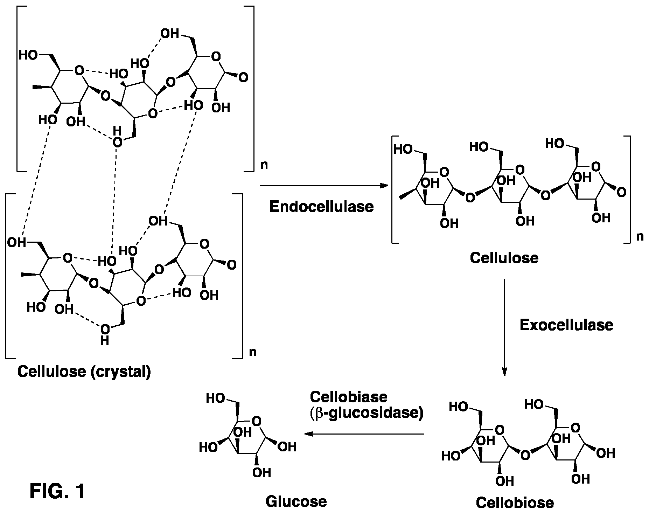

Referring to FIG. 1, during saccharification, a cellulosic or lignocellulosic substrate is initially hydrolyzed by endoglucanases at random locations producing oligomeric intermediates. These intermediates are then substrates for exo-splitting glucanases such as cellobiohydrolase to produce cellobiose from the ends of the cellulose polymer. Cellobiose is a water-soluble 1,4-linked dimer of glucose. Finally cellobiase cleaves cellobiose to yield glucose. The glucose, or other sugars, derived from saccharification can be utilized in a fuel cell as will be described in detail herein.

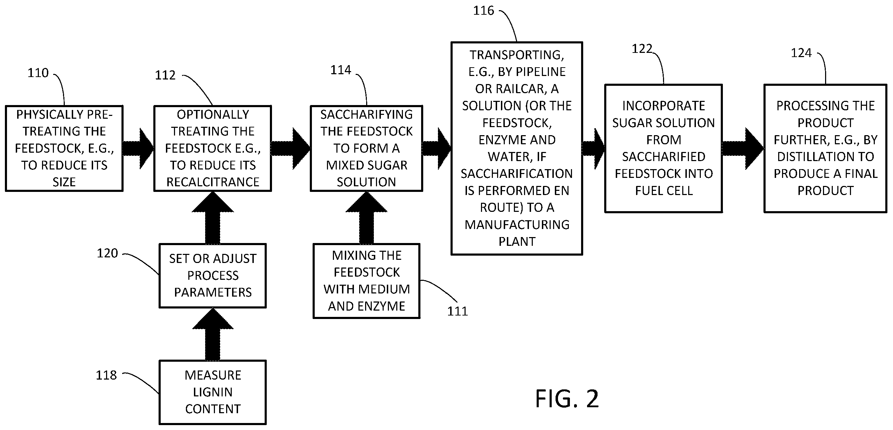

Referring to FIG. 2, a process for manufacturing sugar solutions for incorporation into a fuel cell system can include, for example, optionally mechanically treating a cellulosic and/or lignocellulosic feedstock (step 110). Before and/or after this treatment, the feedstock can optionally be treated with another physical treatment, for example irradiation, to reduce or further reduce its recalcitrance (step 112). A sugar solution is formed by saccharifying the feedstock (step 114) by, for example, the addition of one or more enzymes (step 111). Optionally, the method may also include transporting, e.g., by pipeline, railcar, truck or barge, the sugar solution (or the feedstock, enzyme and water, if saccharification is performed en route) to a manufacturing plant (step 116). For example, the methods of transporting and processing biomass as discussed in U.S. Patent 8,318,453 filed Jan 21, 2009 can be used herein. If desired, the steps of measuring lignin content (step 118) and setting or adjusting process parameters based on this measurement (step 120) can be performed at various stages of the process, for example, as described in U.S. Application Number 12/704,519, filed on February 11, 2011. The sugar solution is then incorporated into a fuel cell or fuel cell system (122). Optionally, products produced in the process can be further processed and/or modified, for example, if sugars from the process are fermented to products, the product can be purified, for example by distillation (124). The products produced in these processes can also be utilized in a fuel cell system.

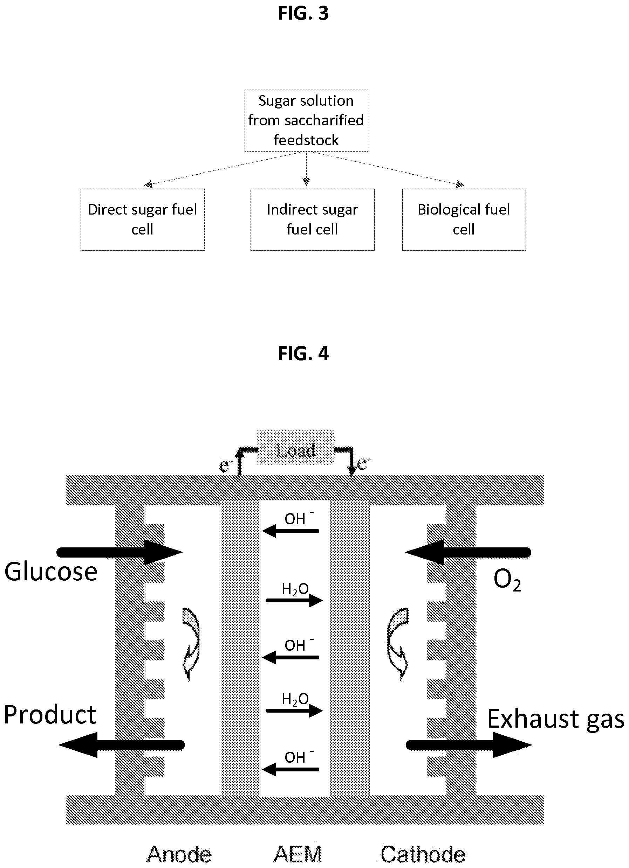

FIG. 3 is a diagram showing processes using saccharified feedstock in fuel cells. The sugar solution from the saccharified feedstock can be used in a direct sugar fuel cell, an indirect sugar fuel cell or a biological fuel cell. Glucose and xylose are often the most abundant sugars available from biomass and sugar solutions derived from biomass can include a mixture of xylose and glucose in various ratios. For example, only glucose can be present or only xylose can be present, especially in cases when the sugars have been isolated and/or purified. When desired, other ratios can be utilized, for example as a percent of total glucose and xylose, the amount of glucose can be between 100% and 90%, 90% and 80%, 80% and 70%, 70% and 60%, 60% and 50%, 50% and 40%, 40% and 30%, 30% and 20%, 20% and 10%, 10% and 0%. Although, the glucose and xylose are often abundant biomass derived sugars, often providing more than 10 wt. % of the sugar to biomass (e.g., more than 20 wt.%, more than 30 wt.%, more than 40 wt.%, more than 50 wt.%, more than 60 wt.%, or even more than 70 wt.%) and are useful in these different fuel cells, other sugars and polysaccharides can also be useful. For example, arabinose, mannose, galactose and rhamnose, cellulose, starch, xylan, glucuronoxylan, arabinoxylan, glucomannan and xylogulcan can be used. Mixtures of any of these sugars can be utilized. In addition the sugars described herein can be isomerized (e.g., to fructose) and then used in a fuel cell. These different fuel cells and their use are discussed in more detail below.

Direct sugar fuel cells, for example glucose fuel cells, generally include a cathode electrode, an anode electrode, and a separator (e.g., an anion-exchange membrane (AEM) or a diffusion layer.) The fuel cell may be acidic or alkaline. In the example shown in FIG. 4, an AEM is sandwiched between an anode electrode and a cathode electrode, with flow fields being provided between each of the electrodes and the AEM. In some cases, the cell has a two-cylinder construction in which one electrode (e.g., the anode) is in the form of an inner cylinder and the other (e.g., the cathode) is in the form of an outer cylinder.

The active component of the anode may be, for example, PdNi or Pd-Pt, and the active component of the cathode may be, for example, a combined catalyst of cobalt porphyrin complex (CoTPP) and spinel (MnCo₂O₄) or other suitable catalyst.

In the embodiment of a direct sugar fuel cell shown in FIG. 4, a fuel solution containing glucose and, generally, potassium hydroxide (KOH), is fed into the anode flow channel, e.g., by a peristaltic pump (not shown), while oxygen is fed to the cathode flow field. Glucose is oxidized at the anode and the reduced product flows away through an anode exit channel. The electrons flow from the anode and through a load. Oxygen is reduced at the cathode and exhaust gas is vented from the cathode flow field.

Direct sugar fuel cells may or may not completely oxidize the sugar fuel to carbon dioxide and water while generating electricity. For example, the reaction for the total oxidation of glucose as shown here may occur.
Anode reaction: C₆H₁₂O₆ +24OH → 18H₂O + 6CO₂ + 18e-
Cathode reaction: 6O₂ + 12H₂O + 24e- → 24OH⁻
Overall reaction: C₆H₁₂O₆ + 6O₂ → 6CO₂ + 6H₂O ΔH = -2830 KJ/mol
Since the total oxidation of glucose proceeds through many intermediates (e.g., gluconic acid, glucaric acid, gamma-gluconolactone, gamma-glucaro lactone, 2-ketogluconic acid, arabinose, trihydroxyglutaric acid, tartaric acid, hydroxyl malonic acid and oxalic acid) any of these intermediates can also be used in a fuel cell. Any of these intermediates, if produced by some of the processed described herein, (e.g., saccharification, fermentation) can be useful in generating electricity in a fuel cell. Optionally, any sugar products not used in generating electricity in the direct sugar fuel cell can be further processed as shown in FIG. 2, for example they can be fermented to an alcohol and the alcohol isolated by distillation. In some cases the fuel cell can use one sugar, for example glucose, and does not use other sugars, for example xylose, and the second sugar can be used in subsequent processes. In some other instances, a process, for example fermentation, only uses one sugar (e.g., glucose) leaving other sugars which can be then used in a fuel cell. In many cases, only a partial oxidation of glucose occurs in a direct sugar fuel cell. For example, the oxidation of glucose to gluconic acid occurs quickly providing 2 electrons and releasing at most about 200 KJ/mol of energy (e.g., about 7% of the available energy). In terms of usable energy, the fuel cells convert at least 1% of the fuel to electric energy (e.g., at least 5%, at least 7%, at least 10%, at least 14%, at least 20%, at least 25%, at least 30 %, at least 40%, at least 50%, at least 60%, at least 70%, at least 80%, at least 90%). In some cases between about 1% and 90% of the fuel is converted to electric energy (e.g., between about 1% and 70%, between about 1% and 50%, between about 1% and 20 %, between about 1% and 10%, between about 5% and 90%, between about 5% and 70%, between about 5% and 50%, between about 5% and 25%, between about 5% and 10%, between about 10% and 90%, between about 10% and 70%, between about 10% and 50%, between about 10% and 30%, between about 10% and 20%, between about 20% and 70%).

In addition to sugars and products such as alcohols, the solutions derived from biomass by the processes described herein can include various solids or dissolved compounds and/or materials. For example the solutions can include enzymes (e.g., parts of enzymes, active enzymes, denatured enzymes), amino acids, nutrients, live cells, dead cells, cellular debris (e.g., lysed cells, yeast extract), acids, bases, salts (e.g., halides, sulfates, and phosphates, alkai, alkali earth, transition metal salts), partial hydrolysis products (e.g., cellulous and hemicellulose fragments), lignin, lignin residues, inorganic solids (e.g., siliceous materials, clays, carbon black, metals), remnants of saccharified biomass and combinations thereof.

Enzymes that can be present can be the intact or denatured enzymes utilized in the processing, or derived from these enzymes (e.g., proteins and amino acids). These can be dissolved/and or precipitated and suspended solids. For example, the sugar solutions can have up to about 10 wt.% enzymes (e.g., up to 9 wt. %, up to 8 wt. %, up to 5 wt. %, up to 2 wt. %, up to 1 wt. %, between about 0.1 and 5 wt. %, between about 1 wt. % and 5 wt. %, between about 2 wt. % and 5 wt. %, between about 0.1 wt. % and 1 wt. %, between about 0.01wt.% and 1 wt.%, between about 0.001 wt.% and 0.1 wt.%).

During saccharification of a biomass, an optimal pH can often be in the acidic region and therefore the solutions used, if used directly in a fuel cell system, can have a pH between about 2 and 5 (e.g., between about 4 and 5). The pH can also be adjusted up or down after saccharification and or saccharification done at a higher or lower pH. In some embodiments the solution used in the fuel cell can therefore have pH values selected from a broad range. For example, the pH can be selected from a range of about 2 to about 10 (e.g., between about 2 and 5, between about 3 and 5, between about 3 and 6, between about 4 and 6, between about 5 and 10, between about 6 and 10 between about 8 and 10).

The sugar solutions derived from the processes described herein and used in fuel cells systems can include non-sugar suspended or dissolved solids present at concentrations up to about 50 wt.%, for example between about 1 and 50 wt.%, 2 and 40 wt. %, 3 and 25 wt.%, 5 and 25 wt.%, 40 and 50 wt.%, 30 and 40 wt.%, 10 and 20 wt.%, 1 and 5 wt.%, 10 and 40 wt.%, less than about 50 wt.%, less than about 40 wt.%, less than about 30 wt.%, less than about 20 wt.%, less than about 10 wt.%, less than about 5 wt.%, less than about 1 wt.%, less than about 0.5 wt.%, less than about 0.01 wt.%. These solutions can have high turbidity, for example at least about 5 nephelometric turbidity units (NTU) (e.g., at least about10 NTU, at least about 50 NTU, at least about 100 NTU, at least about 100 NTU , at least about 200 NTU, at least about 300 NTU, at least about 400 NTU and even greater than about 500 NTU). In some cases the solids are completely or partially removed prior to being use in the fuel cells. For example the solids can be removed by filtration, centrifuging, settling, floatation and combinations of these. In some cases the solids are derived from a previously soluble material that has been precipitated, for example an enzyme that has been denatured. After removing the solids the turbidity of the solutions can be reduced by up to about 500 NTU (e.g., reduced by up to about 100 NTU, reduced by up to about 50 NTU, reduced by up to about 5 NTU).

In addition to being turbid, the sugar solutions produced from the processes described herein can be colored due to colored impurities. For example some metal ions and polyphenols and lignin derived products produced or released during the processing of a lignocellulosic biomass can be highly colored. The solutions can be used directly in the fuel cell systems described herein or can be partially or completely decolorized prior to being used. For example the colored impurities can be filtered out of the solution, destroyed (e.g., by chemical decomposition) and/or precipitated out of the solution.

The ionic strength of the biomass derived sugar solutions can be high due to the source of the biomass as well as the processing of the biomass as described herein. The solutions can be used directly or fully, selectively or partially de-ionized prior to being used in the fuel cell systems described herein.

In some embodiments the fuel cell can include biomass (e.g., lignocellulosic biomass, cellulosic biomass, starch) as well as a saccharifying enzyme. For example, sugar can be utilized in a fuel cell system while it is being produced by the action of a saccharifying enzyme on a biomass material.

In yet other embodiments the sugar solutions used in the fuel cells herein described can include an additive. Such additives can modify properties of the solutions such as the pH, viscosity, chemical potential, surface tension, thermal properties, color, odor, opacity, ionic strength, conductivity, sterility and/or nutrient value. For example additives can be acids, bases, buffers (e.g., phosphate buffers), minerals, colloids, emulsions, emulsifiers, particulates, nano-particles, cations, anions, metal ions (e.g., Fe²⁺' Fe³⁺, Mn2⁺, Cu²⁺, K⁺, Na⁺), vitamins, enzymes, peptones, extracts, surfactants, nutrients, gases (e.g., hydrogen, nitrogen, helium, argon, carbon monoxide, carbon dioxide), chemicals, nitrogen sources (e.g., ammonia, urea), pigments, fragrances, anionic polymers, cationic polymers, non-ionic polymers, oligomers, lipids, fats, surfactants, dispersants, anti-foam agents, bacteriostatic agents, antimicrobial agents, microorganisms, viscosity modifiers, oxidizing agents (e.g., peroxides, chlorates), reducing agents, corrosion inhibitors, precipitating agents, coagulants added in any combination and sequence. Additives can be added in ranges from a few parts per million to several percents. For example 1 to 1000 ppm, 5 to 500 ppm, 5 to 100 ppm, 50 to 100 ppm, 100 to 1000 ppm, 1 to 10 wt.%, 2 to 10 wt.%, 5 to 10 wt.%, 2 to 5 wt.%. In some embodiments including cations, anions, metal anions the amounts are between 1 to 1000ppm. In some embodiments where acids, bases or buffers are added, the final pH after addition of the additive can be chosen to be between pH 2 and 10, (e.g., between about 4 and 8, between about 5 and 7, between about 6 and 8, between about 4 and 5,
between about 7 and 8, between about 8 and 10 and between about 2 and 4). Additives can also be metered and added in amounts between about 1 µM to 5 M amounts (e.g., between about 1 mM and 1 M, between about 5 mM and 100 mM, between about 100 mM and 1 molar, between about 10 mM and 100mM).

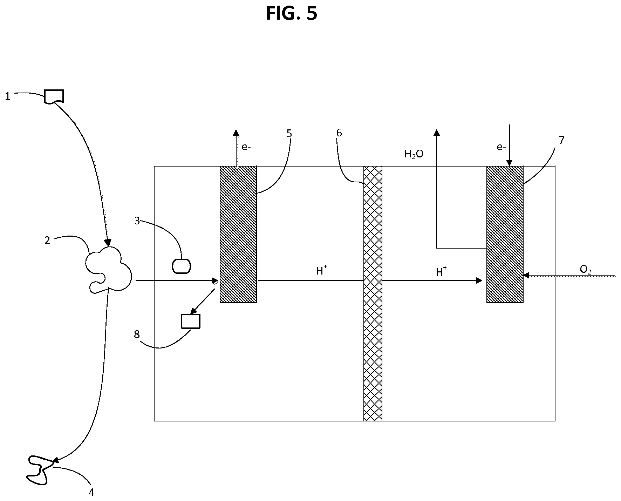

FIG. 5 is a diagrammatic view of an example of an indirect sugar fuel cell. Generally, the indirect sugar fuel cell uses a biological process to convert a primary fuel to a secondary fuel and the secondary fuel generates a current using a fuel cell. The primary fuel (1) is brought into contact with a bio-component (2) where it produces a secondary fuel (3) and waste (4). The secondary fuel is brought into the fuel cell and comes into contact with the anode (5) where it is oxidized, producing a reduced product (8), releasing an electron to an external circuit, and providing a proton. The proton travels in the fuel cell through an ion selective membrane (6) to the cathode (7). Oxygen is supplied to the cathode where it is reduced by electrons supplied from the external circuit and combines with the proton producing water. In another possible design, the bio-component resides within the fuel cell, so that the production of product and electricity all occur within the fuel cell. In some cases, the ion selective membrane is also not required. In other cases the oxidant may be oxidants other than dioxygen (e.g., hydrogen peroxide, organic peroxides and inorganic peroxides).

The primary fuel used in the indirect fuel cell can be sugars (e.g., glucose and xylose) as well as polysaccharides that can be produced through the saccharification of biomass as previously discussed. The secondary fuel can be a fermentation product of the primary fuel. For example, the secondary fuel can be an alcohol or other fermentation product (e.g., ethanol, methanol, butanol, polyols, acetic acid, lactic acid and H₂). Generally, the primary and secondary fuels can be selected from the intermediates and products discussed below. The bio-component can be a microbial material including but are not limited to, any naturally occurring or genetically modified microorganism or organism, for example, protists, *e*.*g*., animal protists (*e*.*g*., protozoa such as flagellates, amoeboids, ciliates, and sporozoa) and plant protists *(e.g.,* algae such alveolates, chlorarachniophytes, cryptomonads, euglenids, glaucophytes, haptophytes, red algae, stramenopiles, and viridaeplantae). Other examples include seaweed, plankton (e.g., macroplankton, mesoplankton, microplankton, nanoplankton, picoplankton, and femptoplankton), phytoplankton, bacteria (e.g., gram positive bacteria, gram negative bacteria, and extremophiles), human cells, mammalian cells, yeast and/or mixtures of these. There may be several bio-components, for example there may be several bacteria specialized to generate different products useful for producing a current from different or the same components of the fuel. For example, fermentation methods and fermenting organisms discussed herein can be utilized to produce the secondary product.

Some species of microorganisms that can be used to produce the secondary fuel in the indirect sugar fuel cell are: *Clostridium saccharobutylacetonicum*, *Clostridium saccharoperbutylacetonicum*, *Clostridium saccharobutylicum*, *Clostridium puniceum, Clostridium beijernckii, Clostridium acetobutylicum, Clostridium aurantibutyricum, Clostridium felsineum, Clostridium butyricum, Geobacter species,* strains of the genus *Sacchromyces spp.* e.g., *Sacchromyces cerevisiae* (baker's yeast), *Saccharomyces distaticus, Saccharomyces uvarum,* strains of the genus *Kluyveromyces*, e.g., species *Kluyveromyces marxianus*, *Kluyveromyces fragilis*, strains of the genus *Candida*, e.g., *Candida pseudotropicalis*, and *Candida brassicae*, *Pichia stipitis* (a relative of *Candida shehatae*), strains of the genus *Clavispora*, e.g., species *Clavispora lusitaniae* and *Clavispora opuntiae*, strains of the genus *Pachysolen*, e.g., species *Pachysolen tannophilus*, and strains of the genus *Bretannomyces*, e.g., species *Bretannomyces clausenii* (Philippidis, G. P., 1996, Cellulose bioconversion technology, in Handbook on Bioethanol: Production and Utilization, Wyman, C.E., ed., Taylor & Francis, Washington, DC, 179-212). Commercially available yeasts the may be used for the formation of secondary fuel include, for example, Red Star®/Lesaffre Ethanol Red (available from Red Star/Lesaffre, USA) FALI® (available from Fleischmann's Yeast, a division of Burns Philip Food Inc., USA), SUPERSTART® (available from Alltech, now Lalemand), GERT STRAND® (available from Gert Strand AB, Sweden) and FERMOL® (available from DSM Specialties).

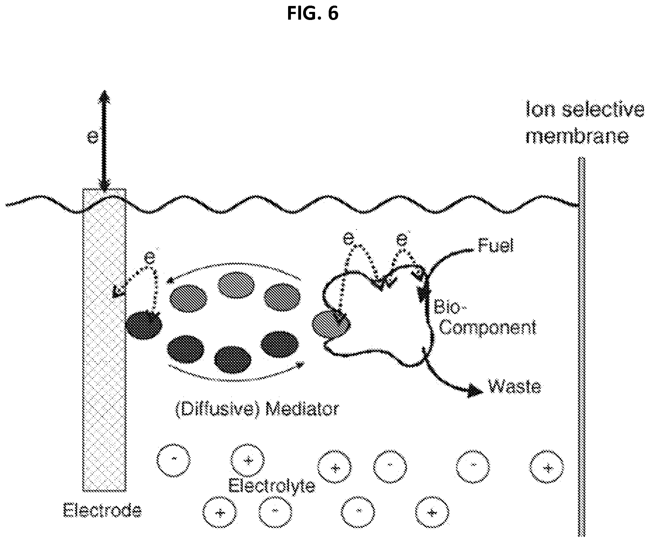

Biological fuel cells are devices capable of directly transforming a chemical fuel to electrical energy via electrochemical reactions involving biochemical pathways. Generally this involves enzymes or active parts of enzymes for catalysis. The enzymes can be within a living organism (e.g., microbial fuel cells) or can be outside of a living cell (e.g., enzyme fuel cells). FIG. 6 shows a diagrammatic view of a generalized half-cell for a biological fuel cell. A supplied fuel is contacted with a biological component which oxidizes the fuel and creates waste that is removed. The electrons released from the fuel are transferred from the biological component to a mediator which either diffuses to or is associated with the electrode where the mediator is oxidized to its original state releasing an electron to an external circuit. The oxidant side of the fuel cell is not shown. Some oxidants can be, for example, O₂, supplied by air, or peroxides (e.g., hydrogen peroxide, organic peroxides, inorganic peroxides). Some biological fuel cells do not require a mediator; in such cells the electron is transferred directly from the biological component to the electrode. In some biological fuel cells the anode reaction with oxygen is catalyzed by a biological component. Some biological fuel cells have been described, for example by Derek Lovely in "The microbe electric: conversion of organic matter to electricity", Current Opinions in Biotechnology, 2008, Volume 19, pages 1-8, or in U.S. patent No. 8,283,076 filed May 18, 2007.

The fuel used in the biological fuel cell can be the saccharification products from biomass as previously discussed. Especially in cases where organisms are utilized (e.g., microbial fuel cells) other nutrients and media components can be added to the fuels, for example ions (e.g., sodium, potassium, iron, magnesium, manganese, zinc, copper), phosphates, sulfates, ammonia, urea, amino acids, proteins, vitamins, buffers, organic acids, inorganic acids, organic bases, inorganic bases, nutrient rich extracts(e.g., yeast extracts, meat extracts and vegetable extracts). Additionally the temperature and pH can be controlled. For example temperatures between 10 and 70 °C can be used (e.g., between about 10 and 60 °C , between about 10 and 50 °C, between about 10 and 40 °C, between about 20 and 70 °C, between about 20 and 60 °C, between about 20 and 50 °C, between about 20 and 40 °C, between about 30 and 70 °C, between about 30 and 60 °C, between about 30 and 50 °C, between about 30 and 40 °C). The pH can be between about 3 and 10 (e.g., between about 3 and 9, between about 3 and 8, between about 3 and 7, between about 3 and 6, between about 3 and 5, between about 4 and 9, between about 4 and 8, between about 4 and 7, between about 5 and 9, between about 5 and 8, between about 5 and 7).

Examples of organisms that can be useful in biological fuel cells are species of geobacter, *proteus vulgaris*, *Desulphovibrio desulphuricans*, *E. coli, Actinobacillus succinogenes*, *Desulphovibrio vulgaris*, Shewanella putrefaciens and *Rhodoferax ferrireducens.*

Examples of enzymes that can be useful in biological fuel cells are glucose oxidase, alcoholdehydrogenase, aldehydedehydrogenase, formate dehydrogenase, oxidoreductase, diaphorase, flavor-oxido-reductase, laccase, microperoxidase, glucose dehydrogenase, hydrogenase, peroxidases, reconstituted enzymes from this list and combinations thereof.

### MECHANICAL TREATMENTS

Biomass feedstock (e.g., cellulosic and/or lignocellulosic material) can be mechanically treated prior to or after other treatments. Methods of mechanically treating the carbohydrate-containing material include, for example, milling or grinding. Milling may be performed using, for example, a hammer mill, ball mill, colloid mill, conical or cone mill, disk mill, edge mill, Wiley mill, grist mill or other mills. Grinding may be performed using, for example, a cutting/impact type grinder. Some exemplary grinders include stone grinders, pin grinders, coffee grinders, and burr grinders. Grinding or milling may be provided, for example, by a reciprocating pin or other element, as is the case in a pin mill. Other mechanical treatment methods include mechanical ripping or tearing, other methods that apply pressure to the fibers, and air attrition milling. Suitable mechanical treatments further include any other technique that continues the disruption of the internal structure of the material that was initiated by the previous processing steps.

Mechanical feed preparation systems can be configured to produce streams with specific characteristics such as, for example, specific maximum sizes, specific length-to-width, or specific surface areas ratios. Physical preparation can increase the rate of reactions, improve the movement of material, improve the irradiation profile of the material, improve the radiation uniformity of the material, or reduce the processing time required by opening up the materials and making them more accessible to processes and/or reagents, such as reagents in a solution.

The bulk density of feedstocks can be controlled (*e*.*g*., increased). In some situations, it can be desirable to prepare a low bulk density material, e.g., by densifying the material (*e*.*g*., densification can make it easier and less costly to transport to another site) and then reverting the material to a lower bulk density state (*e*.*g*., after transport). The material can be densified, for example from less than about 0.2 g/cc to more than about 0.9 g/cc (*e*.*g*., less than about 0.3 to more than about 0.5 g/cc, less than about 0.3 to more than about 0.9 g/cc, less than about 0.5 to more than about 0.9 g/cc, less than about 0.3 to more than about 0.8 g/cc, less than about 0.2 to more than about 0.5 g/cc). For example, the material can be densified by the methods and equipment disclosed in U.S. Pat. No. 7,932,065 to Medoff and International Publication No. WO 2008/073186 (which was filed October 26, 2007, was published in English, and which designated the United States). Densified materials can be processed by any of the methods described herein, or any material processed by any of the methods described herein can be subsequently densified.

In some embodiments, the material to be processed is in the form of a fibrous material that includes fibers provided by shearing a fiber source. For example, the shearing can be performed with a rotary knife cutter.

For example, a fiber source, *e*.*g*., that is recalcitrant or that has had its recalcitrance level reduced, can be sheared, *e*.*g*., in a rotary knife cutter, to provide a first fibrous material. The first fibrous material is passed through a first screen, *e*.*g*., having an average opening size of 1.59 mm or less (1/16 inch, 0.0625 inch), provide a second fibrous material. If desired, the fiber source can be cut prior to the shearing, *e*.*g*., with a shredder. For example, when a paper is used as the fiber source, the paper can be first cut into strips that are, *e*.*g*., 1/4- to 1/2-inch (0.635 to 1.27 cm) wide, using a shredder, *e*.*g*., a counter-rotating screw shredder, such as those manufactured by Munson (Utica, N.Y.). As an alternative to shredding, the paper can be reduced in size by cutting to a desired size using a guillotine cutter. For example, the guillotine cutter can be used to cut the paper into sheets that are, *e*.*g*., 10 inches (25.4 cm) wide by 12 inches (30.48 cm) long.

In some embodiments, the shearing of the fiber source and the passing of the resulting first fibrous material through a first screen are performed concurrently. The shearing and the passing can also be performed in a batch-type process.

For example, a rotary knife cutter can be used to concurrently shear the fiber source and screen the first fibrous material. A rotary knife cutter includes a hopper that can be loaded with a shredded fiber source prepared by shredding a fiber source.

In some implementations, the feedstock is physically treated prior to saccharification and/or fermentation. Physical treatment processes can include one or more of any of those described herein, such as mechanical treatment, chemical treatment, irradiation, sonication, oxidation, pyrolysis or steam explosion. Treatment methods can be used in combinations of two, three, four, or even all of these technologies (in any order). When more than one treatment method is used, the methods can be applied at the same time or at different times. Other processes that change a molecular structure of a biomass feedstock may also be used, alone or in combination with the processes disclosed herein.

Mechanical treatments that may be used, and the characteristics of the mechanically treated feedstocks, are described in further detail in U.S. Serial No. 13/276,192, filed October 18, 2011.

In addition to this size reduction, which can be performed initially and/or later during processing, mechanical treatment can also be advantageous for "opening up," "stressing," breaking or shattering the feedstock materials, making the cellulose of the materials more susceptible to chain scission and/or disruption of crystalline structure during the structural modification treatment.

The biomass can be in a dry form, for example with less than about 35% moisture content (e.g., less than about 20 %, less than about 15 %, less than about 10 % less than about 5 %, less than about 4%, less than about 3 %, less than about 2 % or even less than about 1 %). The biomass can also be delivered in a wet state, for example as a wet solid, a slurry or a suspension with at least about 10 wt% solids (*e*.*g*., at least about 20 wt.%, at least about 30 wt. %, at least about 40 wt.%, at least about 50 wt.%, at least about 60 wt.%, at least about 70 wt.%).

The processes disclosed herein can utilize low bulk density materials, for example cellulosic or lignocellulosic feedstocks that have been physically pretreated to have a bulk density of less than about 0.75 g/cm³, *e*.*g*., less than about 0.7, 0.65, 0.60, 0.50, 0.35, 0.25, 0.20, 0.15, 0.10, 0.05 or less, *e*.*g*., less than about 0.025 g/cm³. Bulk density is determined using ASTM D1895B. Briefly, the method involves filling a measuring cylinder of known volume with a sample and obtaining a weight of the sample. The bulk density is calculated by dividing the weight of the sample in grams by the known volume of the cylinder in cubic centimeters. If desired, low bulk density materials can be densified, for example, by methods described in US. Pat. No. 7,971,809 to Medoff.

In some cases, the biomass can be screened through a mesh or perforated plate with a desired opening size, for example, less than about 6.35 mm (¼ inch, 0.25 inch), (*e*.*g*., less than about 3.18 mm (1/8 inch, 0.125 inch), less than about 1.59 mm (1/16 inch, 0.0625 inch), is less than about 0.79 mm (1/32 inch, 0.03125 inch), *e*.*g*., less than about 0.51 mm (1/50 inch, 0.02000 inch), less than about 0.40 mm (1/64 inch, 0.015625 inch), less than about 0.23 mm (0.009 inch), less than about 0.20 mm (1/128 inch, 0.0078125 inch), less than about 0.18 mm (0.007 inch), less than about 0.13 mm (0.005 inch), or even less than about 0.10 mm (1/256 inch, 0.00390625 inch)).

Screening of biomass material can also be by a manual method, for example by an operator or mechanoid (*e*.*g*., a robot equipped with a color, reflectivity or other sensor) that removes unwanted material. Screening can also be by magnetic screening wherein a magnet is disposed near the conveyed material and the magnetic material is removed magnetically.

Optionally, prior to further processing, the biomass material can be heated, for example by IR radiation, microwaves, combustion (*e*.*g*., gas, coal, oil, biomass), resistive heating and/or inductive heating. Heating can be, for example, for the purpose of drying the material. In the case of drying the material, this can also be facilitated, with or without heating, by the movement of a gas (*e*.*g*., air, oxygen, nitrogen, He, CO₂, Argon) over and/or through the biomass.

Optionally, the biomass can be cooled prior to or after mechanical treatment. Cooling material is described in US Pat. No. 7,900,857 to Medoff.

### RADIATION TREATMENT

In some cases, the feedstock may be irradiated to modify its structure and thereby reduce its recalcitrance. Irradiation may, for example, reduce the average molecular weight of the feedstock, change the crystalline structure of the feedstock, change the functionalization of the feedstock (e.g., by oxidation) and/or increase the surface area and/or porosity of the feedstock.

Various other irradiating devices may be used in the methods disclosed herein, including field ionization sources, electrostatic ion separators, field ionization generators, thermionic emission sources, microwave discharge ion sources, recirculating or static accelerators, dynamic linear accelerators, van de Graaff accelerators, and folded tandem accelerators. Such devices are disclosed, for example, in U.S. Pat. No. 7,931,784 to Medoff.

A beam of electrons can be used as the radiation source. A beam of electrons has the advantages of high dose rates (*e*.*g*., 1, 5, or even 10 Mrad per second), high throughput, less containment, and less confinement equipment. Electron beams can also have high electrical efficiency (*e*.*g*., 80%), allowing for lower energy usage relative to other radiation methods, which can translate into a lower cost of operation and lower greenhouse gas emissions corresponding to the smaller amount of energy used. Electron beams can be generated, e.g., by electrostatic generators, cascade generators, transformer generators, low energy accelerators with a scanning system, low energy accelerators with a linear cathode, linear accelerators, and pulsed accelerators.

Electrons can also be more efficient at causing changes in the molecular structure of carbohydrate-containing materials, for example, by the mechanism of chain scission. In addition, electrons having energies of 0.5-10 MeV can penetrate low density materials, such as the biomass materials described herein, *e*.*g*., materials having a bulk density of less than 0.5 g/cm³, and a depth of 0.3-10 cm. Electrons as an ionizing radiation source can be useful, *e*.*g*., for relatively thin piles, layers or beds of materials, *e*.*g*., less than about 0.5 inch (1.27 cm), *e*.*g*., less than about 0.4 inch (1.016 cm), 0.3 inch (0.762 cm), 0.25 inch (0.635 cm), or less than about 0.1 inch (0.254 cm). In some embodiments, the energy of each electron of the electron beam is from about 0.3 MeV to about 2.0 MeV (million electron volts), *e*.*g*., from about 0.5 MeV to about 1.5 MeV, or from about 0.7 MeV to about 1.25 MeV. Methods of irradiating materials are discussed in U.S. Pat. App. Pub. 2012/01000577 A1, filed October 18, 2011.

Electron beam irradiation devices may be procured commercially from Ion Beam Applications, Louvain-la-Neuve, Belgium or the Titan Corporation, San Diego, CA. Typical electron energies can be 0.5 MeV, 1 MeV, 2 MeV, 4.5 MeV, 7.5 MeV, or 10 MeV. Typical electron beam irradiation device power can be 1 KW, 5 KW, 10 KW, 20 KW, 50 KW, 60 KW, 70 KW, 80 KW, 90 KW, 100 KW, 125 KW, 150 KW, 175 KW, 200 KW, 250 KW, 300 KW, 350 KW, 400 KW, 450 KW, 500 KW, 600 KW, 700 KW, 800 KW, 900 KW or even 1000 KW.

Tradeoffs in considering electron beam irradiation device power specifications include cost to operate, capital costs, depreciation, and device footprint. Tradeoffs in considering exposure dose levels of electron beam irradiation would be energy costs and environment, safety, and health (ESH) concerns. Typically, generators are housed in a vault, *e*.*g*., of lead or concrete, especially for production from X-rays that are generated in the process. Tradeoffs in considering electron energies include energy costs.

The electron beam irradiation device can produce either a fixed beam or a scanning beam. A scanning beam may be advantageous with large scan sweep length and high scan speeds, as this would effectively replace a large, fixed beam width. Further, available sweep widths of 0.5 m, 1 m, 2 m or more are available.

In some embodiments, two or more radiation sources are used, such as two or more ionizing radiation sources. For example, samples can be treated, in any order, with a beam of electrons, followed by gamma radiation and UV light having wavelengths from about 100 nm to about 280 nm. In some embodiments, samples are treated with three ionizing radiation sources, such as a beam of electrons, gamma radiation, and energetic UV light. The biomass is conveyed through the irradiation zone (354 in FIG. 3) where it can be irradiated, for example by electrons. It is generally preferred that the bed of biomass material has a relatively uniform thickness, as previously described, while being irradiated.

Effectiveness of changing the molecular/supermolecular structure and/or reducing the recalcitrance of the carbohydrate-containing biomass depends on the electron energy used and the dose applied, while exposure time depends on the power and dose.

In some embodiments, the irradiating (with any radiation source or a combination of sources) is performed until the material receives a dose of at least about 0.05 Mrad, *e*.*g*., at least about 0.1, 0.25, 0.5, 0.75, 1.0, 2.5, 5.0, 7.5, 10.0, 15, 20, 25, 30, 40, 50, 60, 70, 80, 90, 100, 125, 150, 175, or 200 Mrad. In some embodiments, the irradiating is performed until the material receives a dose of between 0.1 - 100 Mrad, 1 - 200, 5 - 200, 10 - 200, 5 - 150, 50 - 150, 100 - 200, 100 - 150, 80 - 120, 5 - 100, 5 - 50, 5 - 40, 10 - 50, 10 - 75, 15 - 50, 20 - 35 Mrad.

In some embodiments, the irradiating is performed at a dose rate of between 5.0 and 1500.0 kilorads/hour, e.g., between 10.0 and 750.0 kilorads/hour or between 50.0 and 350.0 kilorads/hours. In other embodiments the irradiation is performed at a dose rate of between 10 and 10000 kilorads/hr, between 100 and 1000 kilorad/hr, or between 500 and 1000 kilorads/hr.

In some implementations, it is desirable to cool the material during irradiation. For example, the material can be cooled while it is being conveyed, for example by a screw extruder or other conveying equipment.

Radiation can be applied while the cellulosic and/or lignocellulosic material is exposed to air, oxygen-enriched air, or even oxygen itself, or blanketed by an inert gas such as nitrogen, argon, or helium. When maximum oxidation is desired, an oxidizing environment is utilized, such as air or oxygen and the distance from the radiation source is optimized to maximize reactive gas formation, e.g., ozone and/or oxides of nitrogen.

### SONICATION, PYROLYSIS, OXIDATION, STEAM EXPLOSION

If desired, one or more sonication, pyrolysis, oxidative, or steam explosion processes can be used in addition to or instead of irradiation to reduce the recalcitrance of the feedstock. These processes are described in detail in U.S. Serial No. 12/429,045.

### SACCHARIFICATION

The treated biomass materials can be saccharified, generally by combining the material and a cellulase enzyme in a fluid medium, e.g., an aqueous solution. In some cases, the material is boiled, steeped, or cooked in hot water prior to saccharification, as described in U.S. Pat. App. Pub. 2012/01000577 A1, filed October 18, 2011.

The saccharification process can be partially or completely performed in a tank (*e*.*g*., a tank having a volume of at least 4000, 40,000, or 500,000 L) in a manufacturing plant, and/or can be partially or completely performed in transit, *e*.*g*., in a rail car, tanker truck, or in a supertanker or the hold of a ship. The time required for complete saccharification will depend on the process conditions and the carbohydrate-containing material and enzyme used. If saccharification is performed in a manufacturing plant under controlled conditions, the cellulose may be substantially entirely converted to sugar, *e*.*g*., glucose in about 12-96 hours. If saccharification is performed partially or completely in transit, saccharification may take longer.

It is generally preferred that the tank contents be mixed during saccharification, e.g., using jet mixing as described in International App. No. PCT/US2010/035331, filed May 18, 2010, which was published in English as WO 2010/135380 and designated the United States.

The addition of surfactants can enhance the rate of saccharification. Examples of surfactants include non-ionic surfactants, such as a Tween® 20 or Tween® 80 polyethylene glycol surfactants, ionic surfactants, or amphoteric surfactants.

It is generally preferred that the concentration of the sugar solution resulting from saccharification be relatively high, *e*.*g*., greater than 40%, or greater than 50, 60, 70, 80, 90 or even greater than 95% by weight. Water may be removed, *e*.*g*., by evaporation, to increase the concentration of the sugar solution. This reduces the volume to be shipped, and also inhibits microbial growth in the solution.

Alternatively, sugar solutions of lower concentrations may be used, in which case it may be desirable to add an antimicrobial additive, e.g., a broad spectrum antibiotic, in a low concentration, *e*.*g*., 50 to 150 ppm. Other suitable antibiotics include amphotericin B, ampicillin, chloramphenicol, ciprofloxacin, gentamicin, hygromycin B, kanamycin, neomycin, penicillin, puromycin, streptomycin. Antibiotics will inhibit growth of microorganisms during transport and storage, and can be used at appropriate concentrations, e.g., between 15 and 1000 ppm by weight, e.g., between 25 and 500 ppm, or between 50 and 150 ppm. If desired, an antibiotic can be included even if the sugar concentration is relatively high. Alternatively, other additives with anti-microbial or preservative properties may be used. Preferably the antimicrobial additive(s) are food-grade.

A relatively high concentration solution can be obtained by limiting the amount of water added to the carbohydrate-containing material with the enzyme. The concentration can be controlled, *e*.*g*., by controlling how much saccharification takes place. For example, concentration can be increased by adding more carbohydrate-containing material to the solution. In order to keep the sugar that is being produced in solution, a surfactant can be added, *e*.*g*., one of those discussed above. Solubility can also be increased by increasing the temperature of the solution. For example, the solution can be maintained at a temperature of 40-50°C, 60-80°C, or even higher.

### SUGARS

In the processes described herein, for example after saccharification, sugars (*e*.*g*., glucose and xylose) can be isolated. For example sugars can be isolated by precipitation, crystallization, chromatography (*e*.*g*., simulated moving bed chromatography, high pressure chromatography), centrifugation, extraction, any other isolation method known in the art, and combinations thereof.

### FERMENTATION

Yeast and *Zymomonas* bacteria, for example, can be used for fermentation or conversion of sugar(s) to alcohol(s). Other microorganisms are discussed below. The optimum pH for fermentations is about pH 4 to 7. For example, the optimum pH for yeast is from about pH 4 to 5, while the optimum pH for *Zymomonas* is from about pH 5 to 6. Typical fermentation times are about 24 to 168 hours *(e.g.,* 24 to 96 hrs) with temperatures in the range of 20°C to 40°C (*e*.*g*., 26°C to 40°C), however thermophilic microorganisms prefer higher temperatures.

In some embodiments, e.g., when anaerobic organisms are used, at least a portion of the fermentation is conducted in the absence of oxygen, e.g., under a blanket of an inert gas such as N₂, Ar, He, CO₂ or mixtures thereof. Additionally, the mixture may have a constant purge of an inert gas flowing through the tank during part of or all of the fermentation. In some cases, anaerobic condition can be achieved or maintained by carbon dioxide production during the fermentation and no additional inert gas is needed.

In some embodiments, all or a portion of the fermentation process can be interrupted before the low molecular weight sugar is completely converted to a product (*e*.*g*., ethanol). The intermediate fermentation products include sugar and carbohydrates in high concentrations. The sugars and carbohydrates can be isolated via any means known in the art. These intermediate fermentation products can be used in preparation of food for human or animal consumption. Additionally or alternatively, the intermediate fermentation products can be ground to a fine particle size in a stainless-steel laboratory mill to produce a flour-like substance.

Jet mixing may be used during fermentation, and in some cases saccharification and fermentation are performed in the same tank.

Nutrients for the microorganisms may be added during saccharification and/or fermentation, for example the food-based nutrient packages described in U.S. Pat. App. Pub. 2012/0052536, filed July 15, 2011.

Mobile fermenters can be utilized, as described in International App. No. PCT/US2007/074028 (which was filed July 20, 2007, was published in English as WO 2008/011598 and designated the United States). Similarly, the saccharification equipment can be mobile. Further, saccharification and/or fermentation may be performed in part or entirely during transit.

### DISTILLATION

After fermentation, the resulting fluids can be distilled using, for example, a "beer column" to separate ethanol and other alcohols from the majority of water and residual solids. The vapor exiting the beer column can be, *e*.*g*., 35% by weight ethanol and can be fed to a rectification column. A mixture of nearly azeotropic (92.5%) ethanol and water from the rectification column can be purified to pure (99.5%) ethanol using vapor-phase molecular sieves. The beer column bottoms can be sent to the first effect of a three-effect evaporator. The rectification column reflux condenser can provide heat for this first effect. After the first effect, solids can be separated using a centrifuge and dried in a rotary dryer. A portion (25%) of the centrifuge effluent can be recycled to fermentation and the rest sent to the second and third evaporator effects. Most of the evaporator condensate can be returned to the process as fairly clean condensate with a small portion split off to waste water treatment to prevent build-up of low-boiling compounds.

### INTERMEDIATES AND PRODUCTS

Using the processes described herein, the biomass material can be converted to one or more products, such as energy, fuels, foods and materials. Specific examples of products include, but are not limited to, hydrogen, sugars (e.g., glucose, xylose, arabinose, mannose, galactose, fructose, disaccharides, oligosaccharides and polysaccharides), alcohols (*e*.*g*., monohydric alcohols or dihydric alcohols, such as ethanol, n-propanol, isobutanol, *sec*-butanol, tert-butanol or n-butanol), hydrated or hydrous alcohols (*e*.*g*., containing greater than 10%, 20%, 30% or even greater than 40% water), biodiesel, organic acids, hydrocarbons (*e*.*g*., methane, ethane, propane, isobutene, pentane, n-hexane, biodiesel, bio-gasoline and mixtures thereof), coproducts (*e*.*g*., proteins, such as cellulolytic proteins (enzymes) or single cell proteins), and mixtures of any of these in any combination or relative concentration, and optionally in combination with any additives (*e*.*g*., fuel additives). Other examples include carboxylic acids, salts of a carboxylic acid, a mixture of carboxylic acids and salts of carboxylic acids and esters of carboxylic acids (e.g., methyl, ethyl and n-propyl esters), ketones *(e.g.,* acetone), aldehydes *(e.g.,* acetaldehyde), alpha and beta unsaturated acids *(e.g.,* acrylic acid) and olefins *(e.g.,* ethylene). Other alcohols and alcohol derivatives include propanol, propylene glycol, 1,4-butanediol, 1,3-propanediol, sugar alcohols (e.g., erythritol, glycol, glycerol, sorbitol threitol, arabitol, ribitol, mannitol, dulcitol, fucitol, iditol, isomalt, maltitol, lactitol, xylitol and other polyols), and methyl or ethyl esters of any of these alcohols. Other products include methyl acrylate, methylmethacrylate, lactic acid, citric acid, formic acid, acetic acid, propionic acid, butyric acid, succinic acid, valeric acid, caproic acid, 3-hydroxypropionic acid, palmitic acid, stearic acid, oxalic acid, malonic acid, glutaric acid, oleic acid, linoleic acid, glycolic acid, gamma-hydroxybutyric acid, and mixtures thereof, salts of any of these acids, mixtures of any of the acids and their respective salts. Many of the products obtained, such as ethanol or n-butanol, can be utilized as a fuel for powering cars, trucks, tractors, ships or trains, *e*.*g*., as an internal combustion fuel or as a fuel cell feedstock. Many of the products obtained can also be utilized to power aircraft, such as planes, *e*.*g*., having jet engines or helicopters. In addition, the products described herein can be utilized for electrical power generation, *e*.*g*., in a conventional steam generating plant or in a fuel cell plant.

Other intermediates and products, including food and pharmaceutical products, are described in U.S. App. No. 12/417,900 filed April 3, 2009.

### CARBOHYDRATE CONTAINING MATERIALS (BIOMASS MATERIALS)

As used herein, the term "biomass materials" is used interchangeably with the term "carbohydrate-containing materials", and includes lignocellulosic, cellulosic, starchy, and microbial materials. Any of the methods described herein can be practiced with mixtures of any biomass materials described herein.

Lignocellulosic materials include, but are not limited to, wood, particle board, forestry wastes (*e*.*g*., sawdust, aspen wood, wood chips), grasses, (*e*.*g*., switchgrass, miscanthus, cord grass, reed canary grass), grain residues, (*e*.*g*., rice hulls, oat hulls, wheat chaff, barley hulls), agricultural waste (*e*.*g*., silage, canola straw, wheat straw, barley straw, oat straw, rice straw, jute, hemp, flax, bamboo, sisal, abaca, corn cobs, corn stover, soybean stover, corn fiber, alfalfa, hay, coconut hair), sugar processing residues (e.g., bagasse, beet pulp, agave bagasse), algae, seaweed, manure, sewage, and mixtures of any of these.

In some cases, the lignocellulosic material includes corncobs. Ground or hammermilled corncobs can be spread in a layer of relatively uniform thickness for irradiation, and after irradiation are easy to disperse in the medium for further processing. To facilitate harvest and collection, in some cases the entire corn plant is used, including the corn stalk, corn kernels, and in some cases even the root system of the plant.

Advantageously, no additional nutrients (other than a nitrogen source, e.g., urea or ammonia) are required during fermentation of corncobs or cellulosic or lignocellulosic materials containing significant amounts of corncobs.

Corncobs, before and after comminution, are also easier to convey and disperse, and have a lesser tendency to form explosive mixtures in air than other cellulosic or lignocellulosic materials such as hay and grasses.

Cellulosic materials include, for example, paper, paper products, paper waste, paper pulp, pigmented papers, loaded papers, coated papers, filled papers, magazines, printed matter (e.g., books, catalogs, manuals, labels, calendars, greeting cards, brochures, prospectuses, newsprint), printer paper, polycoated paper, card stock, cardboard, paperboard, materials having a high α-cellulose content such as cotton, and mixtures of any of these. For example paper products as described in U.S. App. No. 13/396,365 ("Magazine Feedstocks" by Medoff *et al*., filed February 14, 2012), the full disclosure of which is incorporated herein by reference.

Cellulosic materials can also include lignocellulosic materials which have been de-lignified.

Starchy materials include starch itself, *e*.*g*., corn starch, wheat starch, potato starch or rice starch, a derivative of starch, or a material that includes starch, such as an edible food product or a crop. For example, the starchy material can be arracacha, buckwheat, banana, barley, cassava, kudzu, oca, sago, sorghum, regular household potatoes, sweet potato, taro, yams, or one or more beans, such as favas, lentils or peas. Blends of any two or more starchy materials are also starchy materials. Mixtures of starchy, cellulosic and or lignocellulosic materials can also be used. For example, a biomass can be an entire plant, a part of a plant or different parts of a plant, *e*.*g*., a wheat plant, cotton plant, a corn plant, rice plant or a tree. The starchy materials can be treated by any of the methods described herein.

Microbial materials include, but are not limited to, any naturally occurring or genetically modified microorganism or organism that contains or is capable of providing a source of carbohydrates *(e.g.,* cellulose), for example, protists, *e*.*g*., animal protists (e.g., protozoa such as flagellates, amoeboids, ciliates, and sporozoa) and plant protists (e.g., algae such alveolates, chlorarachniophytes, cryptomonads, euglenids, glaucophytes, haptophytes, red algae, stramenopiles, and viridaeplantae). Other examples include seaweed, plankton (e.g., macroplankton, mesoplankton, microplankton, nanoplankton, picoplankton, and femptoplankton), phytoplankton, bacteria (*e*.*g*., gram positive bacteria, gram negative bacteria, and extremophiles), yeast and/or mixtures of these. In some instances, microbial biomass can be obtained from natural sources, *e*.*g*., the ocean, lakes, bodies of water, *e*.*g*., salt water or fresh water, or on land. Alternatively or in addition, microbial biomass can be obtained from culture systems, e.g., large scale dry and wet culture and fermentation systems.

In other embodiments, the biomass materials, such as cellulosic, starchy and lignocellulosic feedstock materials, can be obtained from transgenic microorganisms and plants that have been modified with respect to a wild type variety. Such modifications may be, for example, through the iterative steps of selection and breeding to obtain desired traits in a plant. Furthermore, the plants can have had genetic material removed, modified, silenced and/or added with respect to the wild type variety. For example, genetically modified plants can be produced by recombinant DNA methods, where genetic modifications include introducing or modifying specific genes from parental varieties, or, for example, by using transgenic breeding wherein a specific gene or genes are introduced to a plant from a different species of plant and/or bacteria. Another way to create genetic variation is through mutation breeding wherein new alleles are artificially created from endogenous genes. The artificial genes can be created by a variety of ways including treating the plant or seeds with, for example, chemical mutagens (*e*.*g*., using alkylating agents, epoxides, alkaloids, peroxides, formaldehyde), irradiation (*e*.*g*., X-rays, gamma rays, neutrons, beta particles, alpha particles, protons, deuterons, UV radiation) and temperature shocking or other external stressing and subsequent selection techniques. Other methods of providing modified genes is through error prone PCR and DNA shuffling followed by insertion of the desired modified DNA into the desired plant or seed. Methods of introducing the desired genetic variation in the seed or plant include, for example, the use of a bacterial carrier, biolistics, calcium phosphate precipitation, electroporation, gene splicing, gene silencing, lipofection, microinjection and viral carriers. Additional genetically modified materials have been described in U.S. Application Serial No 13/396,369 filed February 14, 2012.

### SACCHARIFYING AGENTS

Suitable cellulolytic enzymes include cellulases from species in the genera *Bacillus, Coprinus, Myceliophthora, Cephalosporium, Scytalidium*, *Penicillium, Aspergillus, Pseudomonas, Humicola, Fusarium, Thielavia, Acremonium, Chrysosporium* and *Trichoderma*, especially those produced by a strain selected from the species *Aspergillus* (see, *e.g.,* EP Pub. No. 0 458 162), *Humicola insolens* (reclassified as *Scytalidium thermophilum*, see, *e.g.,* U.S. Pat. No. 4,435,307), *Coprinus cinereus*, *Fusarium oxysporum, Myceliophthora thermophila, Meripilus giganteus*, *Thielavia terrestris*, *Acremonium sp.* (including, but not limited to, *A. persicinum*, *A. acremonium, A. brachypenium*, *A. dichromosporum*, *A. obclavatum*, *A. pinkertoniae*, *A. roseogriseum*, *A*. *incoloratum*, and *A*. *furatum*). Preferred strains include *Humicola insolens* DSM 1800, *Fusarium oxysporum* DSM 2672, *Myceliophthora thermophila* CBS 117.65, *Cephalosporium sp.* RYM-202, *Acremonium sp.* CBS 478.94, *Acremonium sp.* CBS 265.95, *Acremonium persicinum* CBS 169.65, *Acremonium acremonium* AHU 9519, *Cephalosporium sp.* CBS 535.71, *Acremonium brachypenium* CBS 866.73, *Acremonium dichromosporum* CBS 683.73, *Acremonium obclavatum* CBS 311.74, *Acremonium pinkertoniae* CBS 157.70, *Acremonium roseogriseum* CBS 134.56, *Acremonium incoloratum* CBS 146.62, and *Acremonium furatum* CBS 299.70H. Cellulolytic enzymes may also be obtained from *Chrysosporium*, preferably a strain of *Chrysosporium lucknowense*. Additional strains that can be used include, but are not limited to, *Trichoderma* (particularly *T. viride*, *T*. *reesei*, and *T*. *koningii*), alkalophilic *Bacillus* (see, for example, U.S. Pat. No. 3,844,890 and EP Pub. No. 0 458 162), and *Streptomyces* (see, *e*.*g*., EP Pub. No. 0 458 162).

### FERMENTATION AGENTS

The microorganism(s) used in fermentation can be naturally-occurring microorganisms and/or engineered microorganisms. These fermentation agents can be used, for example, to convert a primary fuel to a secondary fuel to be used for energy generation in an indirect fuel cell. Of the fermentation agents can be used to convert sugars or intermediates not used in fuel cells described in the methods herein. Examples of microorganism can be a bacterium (including, but not limited to, *e*.*g*., a cellulolytic bacterium), a fungus, (including, but not limited to, *e*.*g*., a yeast), a plant, a protist, *e*.*g*., a protozoa or a fungus-like protest (including, but not limited to, *e*.*g*., a slime mold), or an alga. When the organisms are compatible, mixtures of organisms can be utilized.

Suitable fermenting microorganisms have the ability to convert carbohydrates, such as glucose, fructose, xylose, arabinose, mannose, galactose, oligosaccharides or polysaccharides into fermentation products. Fermenting microorganisms include strains of the genus *Sacchromyces spp.* (including, but not limited to, *S. cerevisiae* (baker's yeast), *S*. *distaticus*, *S*. *uvarum*), the genus *Kluyveromyces*, (including, but not limited to, *K*. *marxianus*, *K*.*fragilis*), the genus *Candida* (including, but not limited to, *C*. *pseudotropicalis*, and *C*. *brassicae*), *Pichia stipitis* (a relative of *Candida shehatae*), the genus *Clavispora* (including, but not limited to, *C*. *lusitaniae* and *C*. *opuntiae*), the genus *Pachysolen* (including, but not limited to, *P. tannophilus*), the genus *Bretannomyces* (including, but not limited to, *e*.*g*., *B. clausenii* (Philippidis, G. P., 1996, Cellulose bioconversion technology, in Handbook on Bioethanol: Production and Utilization, Wyman, C.E., ed., Taylor & Francis, Washington, DC, 179-212)). Other suitable microorganisms include, for example, *Zymomonas mobilis*, *Clostridium spp.* (including, but not limited to, *Clostridium thermocellum* (Philippidis, 1996, *supra*), *Clostridium saccharobutylacetonicum*, *Clostridium felsineum*, *Clostridium saccharobutylicum*, *Clostridium Puniceum*, *Clostridium beijernckii*, *Clostridium acetobutylicum*, and *Clostridium aurantibutylicum*), *Moniliella pollinis*, *Yarrowia lipolytica*, *Aureobasidium sp., Trichosporonoides sp., Trigonopsis variabilis*, *Trichosporon sp., Moniliellaacetoabutans sp*., *Typhula variabilis*, *Candida magnoliae*, *Ustilaginomycetes sp*., *Pseudozyma tsukubaensis*, yeast species of genera *Zygosaccharomyces*, *Debaryomyces*, *Hansenula and Pichia*, and fungi of the dematioid genus *Torula.*

Many such microbial strains are publicly available, either commercially or through depositories such as the ATCC (American Type Culture Collection, Manassas, Virginia, USA), the NRRL (Agricultural Research Sevice Culture Collection, Peoria, Illinois, USA), or the DSMZ (Deutsche Sammlung von Mikroorganismen und Zellkulturen GmbH, Braunschweig, Germany), to name a few.

Commercially available yeasts include, for example, Red Star®/Lesaffre Ethanol Red (available from Red Star/Lesaffre, USA), FALI® (available from Fleischmann's Yeast, a division of Burns Philip Food Inc., USA), SUPERSTART® (available from Alltech, now Lalemand), GERT STRAND® (available from Gert Strand AB, Sweden) and FERMOL® (available from DSM Specialties).

Other than in the examples herein, or unless otherwise expressly specified, all of the numerical ranges, amounts, values and percentages, such as those for amounts of materials, elemental contents, times and temperatures of reaction, ratios of amounts, and others, in the following portion of the specification and attached claims may be read as if prefaced by the word "about" even though the term "about" may not expressly appear with the value, amount, or range. Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains error necessarily resulting from the standard deviation found in its underlying respective testing measurements. Furthermore, when numerical ranges are set forth herein, these ranges are inclusive of the recited range end points (i.e., end points may be used). When percentages by weight are used herein, the numerical values reported are relative to the total weight.

Also, it should be understood that any numerical range recited herein is intended to include all sub-ranges subsumed therein. For example, a range of "1 to 10" is intended to include all sub-ranges between (and including) the recited minimum value of 1 and the recited maximum value of 10, that is, having a minimum value equal to or greater than 1 and a maximum value of equal to or less than 10. The terms "one," "a," or "an" as used herein are intended to include "at least one" or "one or more," unless otherwise indicated.

While this invention has been particularly shown and described with references to preferred embodiments thereof, it will be understood by those skilled in the art that various changes in form and details may be made therein without departing from the scope of the invention encompassed by the appended claims.

## Claims

1. A method comprising:
enzymatically saccharifying a cellulosic or lignocellulosic material to form a sugar solution comprising xylose and glucose;
delivering the sugar solution to a microbial fuel cell containing an anti-scale agent or an anti-fouling agent; and
utilizing the sugar solution in the microbial fuel cell.

2. The method of claim 1 wherein, prior to saccharification, the recalcitrance of the cellulosic or lignocellulosic material is reduced relative to that of the cellulosic or lignocellulosic material in its native state.

3. The method of claim 2 wherein the method of reducing the recalcitrance of the material is selected from the group consisting of mechanically treating, chemically treating, sonicating, pyrolyzing, irradiating, oxidizing, steam exploding and combinations thereof.

4. The method of claim 2 wherein the method of reducing recalcitrance of the material comprises irradiation the material with ionizing radiation.

5. The method of claim 4 wherein the ionizing radiation comprises electron beam irradiation.

6. The method of claim 4 or 5 wherein the irradiation comprises irradiation with a dose of at least 10 Mrad.

7. The method of claim 1 wherein the cellulosic or lignocellulosic biomass is selected from the group consisting of: paper, paper products, paper waste, paper pulp, pigmented papers, loaded papers, coated papers, filled papers, magazines, printed matter, printer paper, polycoated paper, card stock, cardboard, paperboard, offal, cotton, wood, particle board, forestry wastes, sawdust, aspen wood, wood chips, grasses, switchgrass, miscanthus, cord grass, reed canary grass, grain residues, rice hulls, oat hulls, wheat chaff, barley hulls, agricultural waste, silage, canola straw, wheat straw, barley straw, oat straw, rice straw, jute, hemp, flax, bamboo, sisal, abaca, corn cobs, corn stover, soybean stover, corn fiber, alfalfa, hay, coconut hair, sugar processing residues, bagasse, beet pulp, agave bagasse, algae, seaweed, manure, sewage, arracacha, buckwheat, banana, barley, cassava, kudzu, oca, sago, sorghum, potato, sweet potato, taro, yams, beans, favas, lentils, peas, and mixtures of any of these.

8. The method of claim 1, further comprising mechanically treating the cellulosic or lignocellulosic material, for example, to reduce the bulk density of the material and/or increase its surface area.

9. The method of claim 1 further comprising utilizing an additive in the fuel cell.

10. The method of claim 1, wherein said method is for producing electricity.

11. The method of claim 10 wherein the treatment of the lignocellulosic material is selected from the group consisting of sonication, pyrolysis, irradiation, oxidation, steam explosion and combinations thereof.

12. The method of claim 10 wherein the enzyme used to enzymatically saccharify the treated lignocellulosic material is a cellulase.

13. The method of claim 10 further comprising fermenting the saccharified treated lignocellulosic material to produce a fuel.

14. The method of Claim 13, wherein the fuel is an alcohol.

15. The method of claim 13 or 14 wherein treating the lignocellulosic material comprise irradiation of the lignocellulosic material with ionizing radiation and wherein, optionally, ionizing radiation comprises electron beam irradiation and/or wherein, optionally, irradiation comprises irradiation of the material with a dose of at least 10 Mrad.

## Patentansprüche

1. Verfahren, umfassend:
enzymatisches Verzuckern eines cellulosischen oder lignocellulosischen Materials, um eine Zuckerlösung zu bilden, die Xylose und Glucose umfasst;
Zuführen der Zuckerlösung zu einer mikrobiellen Brennstoffzelle, die ein Entkalkungsmittel oder ein Antifouling-Mittel enthält; und
Verwenden der Zuckerlösung in der mikrobiellen Brennstoffzelle.

2. Verfahren nach Anspruch 1, wobei vor der Verzuckerung die Rekalzitranz des cellulosischen oder lignocellulosischen Materials gegenüber der des cellulosischen oder lignocellulosischen Materials in seinem nativen Zustand reduziert wird.

3. Verfahren nach Anspruch 2, wobei das Verfahren zum Reduzieren der Rekalzitranz des Materials aus der Gruppe bestehend aus mechanischem Behandeln, chemischem Behandeln, Beschallen, Pyrolysieren, Bestrahlen, Oxidieren, Dampfexplosion und Kombinationen davon ausgewählt ist.

4. Verfahren nach Anspruch 2, wobei das Verfahren zum Reduzieren der Rekalzitranz des Materials Bestrahlung des Materials mit ionisierender Strahlung umfasst.

5. Verfahren nach Anspruch 4, wobei ionisierende Strahlung eine Elektronenstrahlbestrahlung umfasst.

6. Verfahren nach Anspruch 4 oder 5, wobei Bestrahlung Bestrahlung mit einer Dosis von mindestens 10 Mrad umfasst.

7. Verfahren nach Anspruch 1, wobei die cellulosische oder lignocellulosische Biomasse ausgewählt ist aus der Gruppe bestehend aus: Papier, Papierprodukten, Papierabfällen, Papierzellstoff, pigmentierten Papieren, beladenen Papieren, beschichteten Papieren, gefüllten Papieren, Zeitschriften, Druckerzeugnissen, Druckerpapier, mehrfach beschichtetem Papier, Karton, Kartonage, Pappe, Schlachtabfall, Baumwolle, Holz, Spanplatten, Forstabfällen, Sägemehl, Espenholz, Holzspänen, Gräsern, Rutenhirse, Miscanthus, Schnurgras, Schilfgras, Kornrückständen, Reishülsen, Haferschalen, Weizenspreu, Gerstenhülsen, landwirtschaftlichen Abfällen, Silage, Rapsstroh, Weizenstroh, Gerstenstroh, Haferstroh, Reisstroh, Jute, Hanf, Flachs, Bambus, Sisal, Abaca, Maiskolben, Maisstroh, Sojabohnenstroh, Maisfaser, Luzerne, Heu, Kokosnusshaar, Zuckerverarbeitungsrückständen, Bagasse, Rübenschnitzel, Agavenbagasse, Algen, Seetang, Dünger, Abwasser, Arakacha, Buchweizen, Banane, Gerste, Maniok, Kopoubohne, Sauerklee, Sago, Sorghum, Kartoffel, Süßkartoffel, Taro, Yams, Bohnen, Favas, Linsen, Erbsen und Mischungen von beliebigen von diesen.

8. Verfahren nach Anspruch 1, ferner umfassend mechanisches Behandeln des cellulosischen oder lignocellulosischen Materials, um beispielsweise die Schüttdichte des Materials zu reduzieren und/oder seine Oberfläche zu vergrößern.

9. Verfahren nach Anspruch 1, ferner umfassend Einsetzen eines Zusatzstoffs in der Brennstoffzelle.

10. Verfahren nach Anspruch 1, wobei das Verfahren zum Erzeugen von Elektrizität dient.

11. Verfahren nach Anspruch 10, wobei die Behandlung des lignocellulosischen Materials aus der Gruppe bestehend aus Beschallung, Pyrolyse, Bestrahlung, Oxidation, Dampfexplosion und Kombinationen davon ausgewählt ist.

12. Verfahren nach Anspruch 10, wobei das Enzym, das zum enzymatischen Verzuckern des behandelten lignocellulosischen Materials verwendet wird, eine Cellulase ist.

13. Verfahren nach Anspruch 10, ferner umfassend Fermentieren des verzuckerten, behandelten lignocellulosischen Materials, um einen Brennstoff zu erzeugen.

14. Verfahren nach Anspruch 13, wobei der Brennstoff ein Alkohol ist.

15. Verfahren nach Anspruch 13 oder 14, wobei Behandeln des lignocellulosischen Materials eine Bestrahlung des lignocellulosischen Materials mit ionisierender Strahlung umfasst und wobei ionisierende Strahlung wahlweise eine Elektronenstrahlbestrahlung umfasst und/oder wobei Bestrahlung wahlweise Bestrahlung des Materials mit einer Dosis von mindestens 10 Mrad umfasst.

## Revendications

1. Procédé comprenant :
la saccarification enzymatique d'une matière cellulosique ou lignocellulosique pour former une solution sucrée comprenant du xylose et du glucose ;
la distribution de la solution sucrée à une pile à combustible microbienne contenant un agent antitartre ou un agent antisalissure ; et
l'utilisation de la solution sucrée dans la pile à combustible microbienne.

2. Procédé selon la revendication 1 dans lequel, avant la saccarification, la récalcitrance de la matière cellulosique ou lignocellulosique est réduite par rapport à celle de la matière cellulosique ou lignocellulosique dans son état naturel.

3. Procédé selon la revendication 2 dans lequel le procédé de réduction de la récalcitrance de la matière est sélectionné dans le groupe constitué d'un traitement mécanique, d'un traitement chimique, de la pyrolyse, d'une irradiation, d'une oxydation, d'une explosion de vapeur et de combinaisons de ceux-ci.

4. Procédé selon la revendication 2 dans lequel le procédé de réduction de la récalcitrance de la matière comprend l'irradiation de la matière avec un rayonnement ionisant.

5. Procédé selon la revendication 4 dans lequel le rayonnement ionisant comprend une irradiation de faisceaux d'électrons.

6. Procédé selon la revendication 4 ou 5 dans lequel l'irradiation comprend une irradiation avec une dose d'au moins 10 Mrad.

7. Procédé selon la revendication 1 dans lequel la biomasse cellulosique ou lignocellulosique est sélectionnée dans le groupe constitué de : papier, produits de papier, déchets de papier, pâte à papier, papiers pigmentés, papiers chargés, papiers enduits, papiers remplis, magazines, imprimés, papier imprimé, papier multicouche, papier fort, carton, papier cartonné, déchets, coton, bois, panneau de particules, déchets forestiers, sciure, bois de tremble, copeaux de bois, herbes, panic érigé, miscanthus, spartine, alpiste roseau, résidus de grain, écorces de riz, écorces d'avoine, balles de blé, écorces d'orge, déchets agricoles, fourrage vert, paille de colza, paille de blé, paille d'orge, paille d'avoine, paille de riz, jute, chanvre, lin, bambou, chanvre de Manille, rafles de maïs, tige de maïs, tige de soja, fibre de maïs, luzerne, foin, poils de noix de coco, résidus de transformation du sucre, bagasse, pulpe de betterave, bagasse d'agave, algue, algue marine, eaux usées, arracacha, sarrasin, banane, orge, manioc, kudzu, oca, sagou, sorgho, pomme de terre, patate douce, taro, ignames, haricots, fèves, lentilles, pois, et mélanges de n'importe lesquels de ceux-ci.

8. Procédé selon la revendication 1, comprenant en outre le traitement mécanique de la matière cellulosique ou lignocellulosique, par exemple, pour réduire la densité apparente de la matière et/ou augmenter sa superficie.

9. Procédé selon la revendication 1 comprenant en outre l'utilisation d'un additif dans la pile à combustible.

10. Procédé selon la revendication 1, dans lequel ledit procédé sert à produire de l'électricité.

11. Procédé selon la revendication 10 dans lequel le traitement de la matière lignocellulosique est sélectionné dans le groupe constitué de la sonification, la pyrolyse, l'irradiation, l'oxydation, l'explosion de vapeur et des combinaisons de celles-ci.

12. Procédé selon la revendication 10 dans lequel l'enzyme utilisée pour la saccarification enzymatique de la matière lignocellulosique traitée est une cellulase.

13. Procédé selon la revendication 10 comprenant en outre la matière lignocellulosique traitée saccarifiée pour produire un combustible, et dans lequel.

14. Procédé selon la revendication 13, dans lequel le combustible est un alcool.

15. Procédé selon la revendication 13 ou 14 dans lequel le traitement de la matière lignocellulosique comprend l'irradiation de la matière lignocellulosique avec un rayonnement ionisant et dans lequel, optionnellement, le rayonnement ionisant comprend une irradiation de faisceaux d'électrons et/ou dans lequel, optionnellement, l'irradiation comprend une irradiation de la matière avec une dose d'au moins 10 Mrad.
